# EUROPEAN PATENT APPLICATION

(11) **EP 4 806 482 A1**
(43) Date of publication of application: **16.09.2026**
(21) Application number: 26164818.2
(22) Date of filing: 13.03.2026
(51) Int. Cl.: A61M 5/32

(54) **DISPOSABLE INJECTION NEEDLE**

(30) Priority: 15.03.2025 CN 202510307084
(71) Applicant: Tianjin Huahong Technology Co., Ltd., Tianjin 300308 (CN)
(72) Inventor: CUI, Chengzhe, Tianjin (CN); YANY, Hao, Tianjin (CN); ZHANG, Libo, Tianjin (CN)
(74) Representative: Bandpay & Greuter

(57) **Abstract**

The present disclosure relates to a disposable injection needle, comprising: a needle seat, a shell, an inner sheath, an inner core, a first spring, a second spring, and a tail sheath.

The tail sheath disposed at the second end of the needle seat and having a tail sheath hole for the needle tube to pass through. wherein the tail sheath comprises an extension member extending substantially in an axial direction, and the tail sheath has a first axial position and a second axial position along the axial extension direction, wherein in the first axial position, the second end of the needle tube is exposed from the tail sheath hole, and in the second axial position, the second end of the needle tube is not exposed from the tail sheath hole. The disposable injection needle provides rear-end protection for the injection needle, thereby preventing the needle tip from being exposed at the rear end of the injection needle, which could cause injury and lead to infection.

## Description

### TECHNICAL FIELD

Embodiments of the present disclosure relate to the field of medical devices, and particularly to a disposable injection needle.

### BACKGROUND

Injection needles for insulin pens, as a tool, have been widely used for their primary function of delivering medication into patients' bodies to achieve therapeutic purposes.

Currently, common injection needles designed for insulin pens on the market consist of a large sheath, a small sheath, a needle seat, a needle tube, and dialysis paper. After removing the large and small sheaths, the needle tip remains exposed, increasing the risk of infection if reused by some patients or potentially causing accidental injuries and cross-contamination.

Most safety insulin needles on the market avoid reuse but only provide front-end protection, leaving the rear end unprotected and still posing safety risks. Existing products offering dual-end protection are overly complex in structure and provide poor user experience.

### SUMMARY

To alleviate or resolve at least one aspect or issue mentioned above, the present disclosure provides a disposable injection needle that achieves dual-end protection for both front and rear ends, featuring a simple structure, high reliability, and excellent user experience.

According to an embodiment of the present disclosure, a disposable injection needle is proposed, comprising:
a needle seat provided with a needle tube extending axially therethrough and having a first end and a second end;
a shell, a second end of the shell being connected to the second end of the needle seat to form an accommodating space between the shell and the needle seat, and a first end of the shell having a second opening;
an inner sheath, a second end of the inner sheath being located within the accommodating space, a first end of the inner sheath having a first opening and being adapted to extend out of the second opening, and a portion of the inner sheath being axially slidable within the accommodating space;
an inner core, a second end of the inner sheath being sleeved on a first end of the inner core, wherein the first end of the inner core comprises a third opening for the first end of the needle tube to extend through;
a second spring disposed between the inner core and the first end of the needle seat and adapted to provide an elastic force for axially moving the inner core and the inner sheath together outward from the accommodating space;
a tail sheath disposed at the second end of the needle seat and having a tail sheath hole for the needle tube to pass through, wherein the tail sheath comprises an extension member extending substantially in an axial direction, and the tail sheath has a first axial position and a second axial position along the axial extension direction, wherein in the first axial position, the second end of the needle tube is exposed from the tail sheath hole, and in the second axial position, the second end of the needle tube is not exposed from the tail sheath hole;
a first spring disposed between the needle seat and the tail sheath to provide power for the tail sheath to move from the first axial position toward the second axial position;
wherein:
   the inner core has a first circumferential position and a second circumferential position, wherein before the injection needle is used, the inner core is in the first circumferential position where the inner core fittingly abuts against the extension member in the axial direction, and after the injection needle is used, the inner core is in the second circumferential position where the inner core is disengaged from the extension member in the axial direction; and
   based on the inner core rotating from the first circumferential position to the second circumferential position, the tail sheath is adapted to move from the first axial position toward the second axial position under the action of the first spring.

### BRIEF DESCRIPTION OF DRAWINGS

FIG. 1 is an exploded perspective view of a disposable injection needle according to an embodiment of the present disclosure.
FIG. 2 is a schematic cross-sectional view of the disposable injection needle shown in FIG. 1.
FIGS. 3 and 4 are perspective views of the inner sheath according to an embodiment of the present disclosure, observed from different angles.
FIGS. 5, 6, and 7 are perspective views of the shell according to an embodiment of the present disclosure, observed from different angles.
FIGS. 8 and 9 are perspective views of the inner core according to an embodiment of the present disclosure, observed from different angles.
FIGS. 10 and 11 are perspective views of the needle seat according to an embodiment of the present disclosure, observed from different angles.
FIGS. 12 and 13 are perspective views of the tail sheath according to an embodiment of the present disclosure, observed from different angles.
FIG. 14 is a schematic diagram of the assembly structure of the inner sheath and the shell according to an embodiment of the present disclosure.
FIG. 15 is a schematic diagram of the assembly of the inner sheath, inner core, and needle seat in the initial state according to an embodiment of the present disclosure.
FIGS. 16 to 19 are schematic diagrams illustrating the fitting state between various components of a disposable injection needle before use, according to an embodiment of the present disclosure.

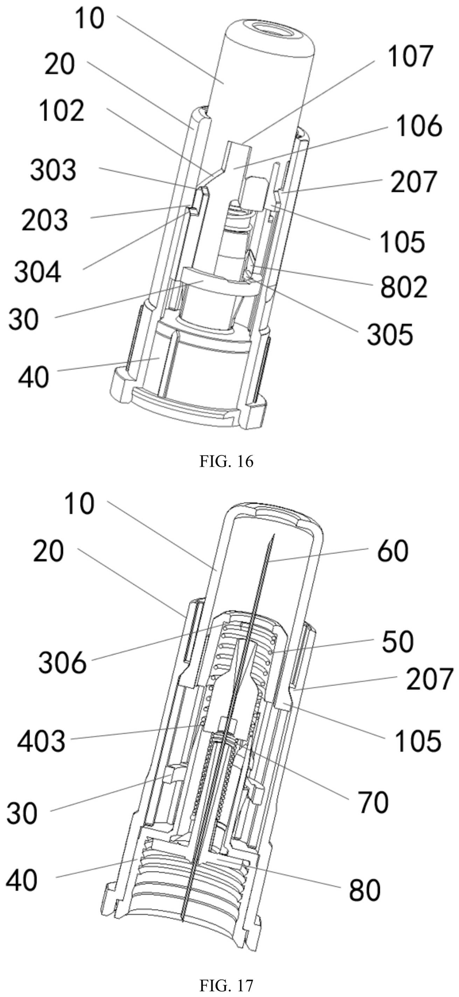

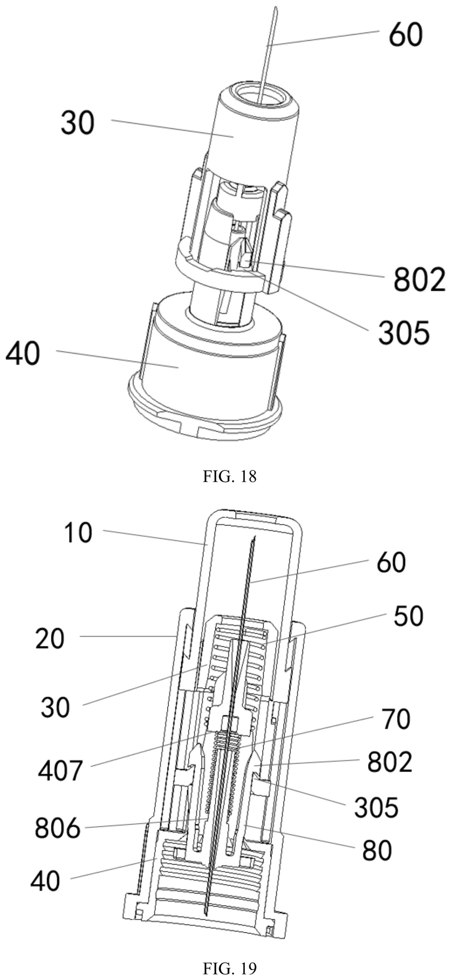

Among them, FIG. 16 shows the positional structure between the first guide part of the inner sheath and the second blocking surface of the shell with the guide mating part of the inner core; FIG. 17 shows the positional structure between the elastic arm end part of the inner sheath and the shell rib; FIGS. 18 and 19 show the positional structure between the inner core engagement part and the tail sheath engagement part.

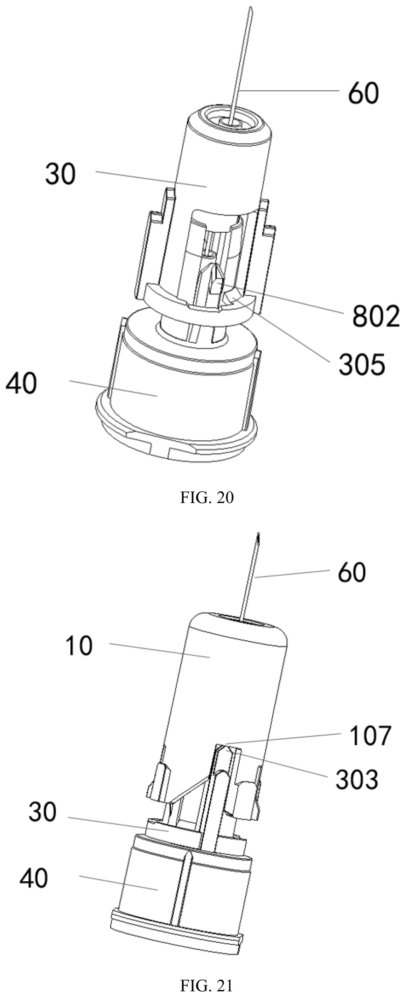

FIG. 20 is a schematic structural diagram of the disposable injection needle during use according to an embodiment of the present disclosure, showing the disengagement between the inner core engagement part and the tail sheath engagement part.

FIG. 21 is a schematic structural diagram of the disposable injection needle during use according to an embodiment of the present disclosure, showing the structural mating state between the inner core and the inner sheath after the inner core rotates.

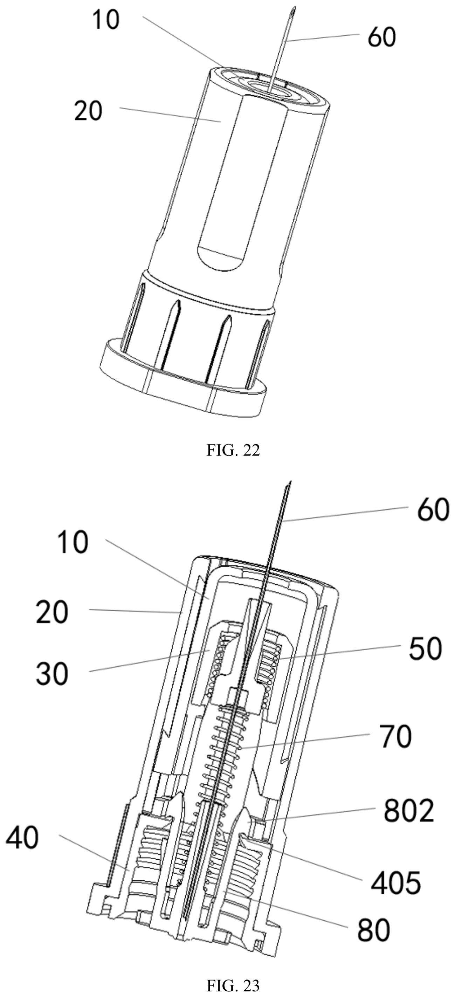

FIG. 22 is a schematic structural diagram of the disposable injection needle during use according to an embodiment of the present disclosure, showing a 3D external structural diagram after the inner sheath is fully pressed down.

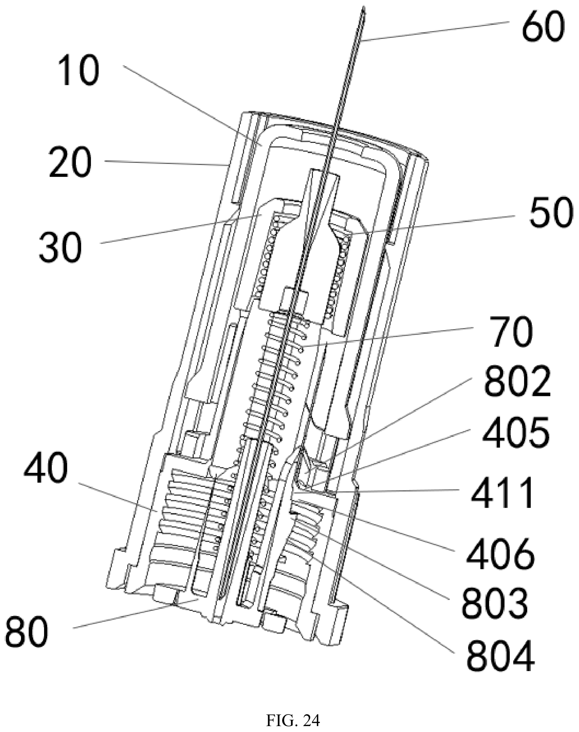

FIGS. 23 and 24 are schematic structural diagrams of the disposable injection needle during use according to an embodiment of the present disclosure, showing the internal structure from different perspectives after the inner sheath is fully pressed down.

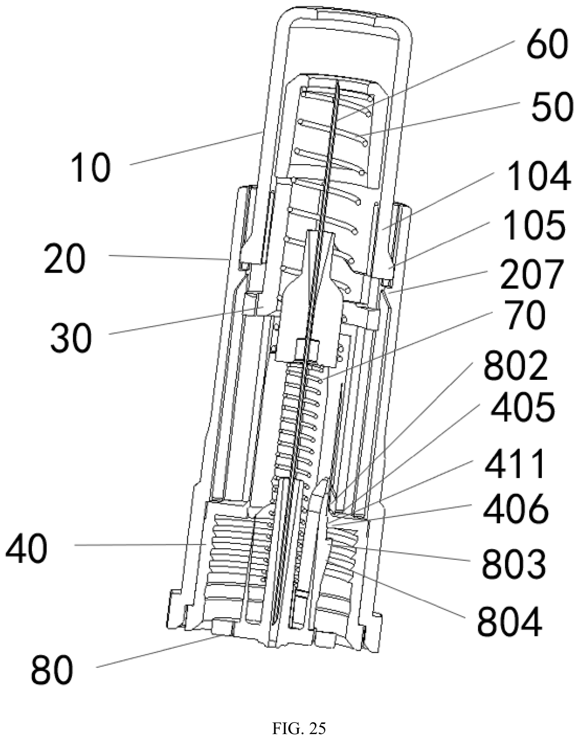

FIGS. 25 to 27 are schematic structural diagrams of the components of the disposable injection needle from different perspectives after use according to an embodiment of the present disclosure.

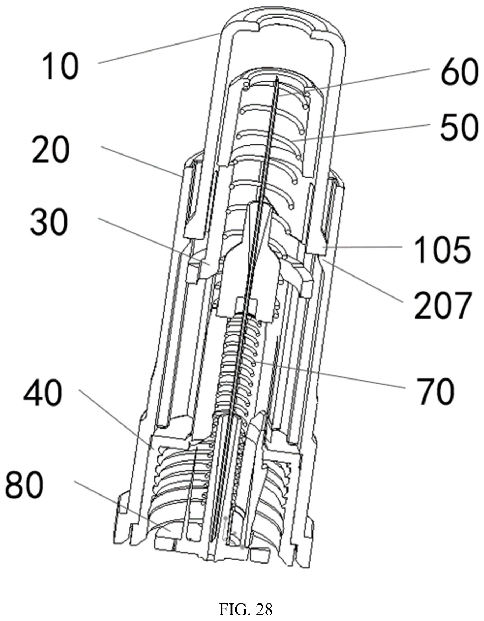

FIG. 28 is a schematic structural diagram of the disposable injection needle when subjected to downward external force again after use according to an embodiment of the present disclosure.

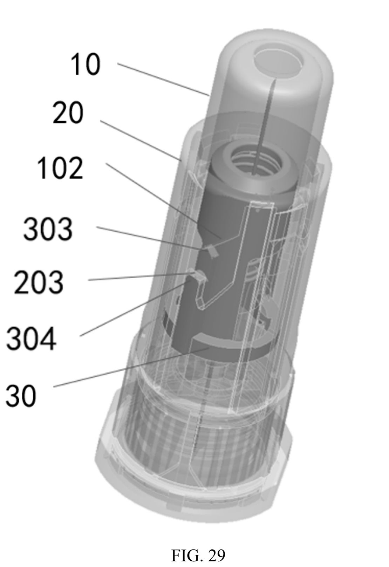

FIG. 29 is a transparent structural view of the components of the disposable injection needle before use according to an embodiment of the present disclosure, showing the positional structure between the first guide part of the inner sheath and the second blocking surface of the shell with the guide mating part of the inner core.

In the figures:
10, Inner sheath;
101, First opening; 102, Inner sheath guide part; 103, Guide block; 104, Elastic arm; 105, Elastic arm end part; 106, First guide groove; 107, First blocking surface;
20, Shell;
201, Second opening; 202, Second guide groove; 203, Second blocking surface; 204, Shell limit rib; 205, Third guide groove; 206, Fourth guide groove; 207, Shell rib; 208, Shell longitudinal slot; 209, Shell transverse slot; 210, Third blocking surface; 211, Shell front end surface;
30, Inner core;
301, Third opening; 302, Inner core guide part; 303, First guide part; 304, Second guide part; 305, Inner core engagement part; 306, Inner core blocking surface; 307, Bottom end surface of the inner core; 308, Inner core front end surface; 309, Inner core through slot; 310, Boss part;
40, Needle seat;
401, Needle seat hole; 402, Small diameter part; 403, Needle seat step surface; 404, Large diameter part; 405, Needle seat limit surface; 406, Bottom surface of needle seat engagement part; 407, Small diameter bottom end surface; 408, Needle seat longitudinal rib; 409, Needle seat transverse rib; 410, Needle seat through slot; 411, Needle seat engagement part;
50, Second spring;
60, Needle tube;
70, First spring;
80, Tail sheath;
801, Extension member; 802, Tail sheath engagement part; 803, Clamping and fitting part; 804, Clamping and fitting inclined surface; 805, Central columnar body; 806, Tail sheath step surface; 807, Tail sheath hole; 808, Tail sheath bottom surface;
90, Outer cover;
100, Sealing label.

### DESCRIPTION OF EMBODIMENTS

The following will clearly and completely describe the technical solutions in the embodiments of the present disclosure with reference to the accompanying drawings. It is evident that the described embodiments are only a part of the embodiments of the present disclosure, not all of them. Based on the embodiments of the present disclosure, all other embodiments obtained by those of ordinary skill in the art without creative effort shall fall within the scope of protection of the present disclosure.

In this disclosure, the terms "upper," "lower," "front," "rear," "top," "bottom," "head," "tail," "inner," and "outer" are illustrative descriptions. In this disclosure, the direction in which the needle tube 60 pierces toward the blood collection site is referred to as the "upper," "front," "head," or "top" direction, and the opposite direction is referred to as the "lower," "rear," "tail," or "bottom" direction. The radial direction closer to the needle tube 60 is referred to as "inner," and the opposite is referred to as "outer."

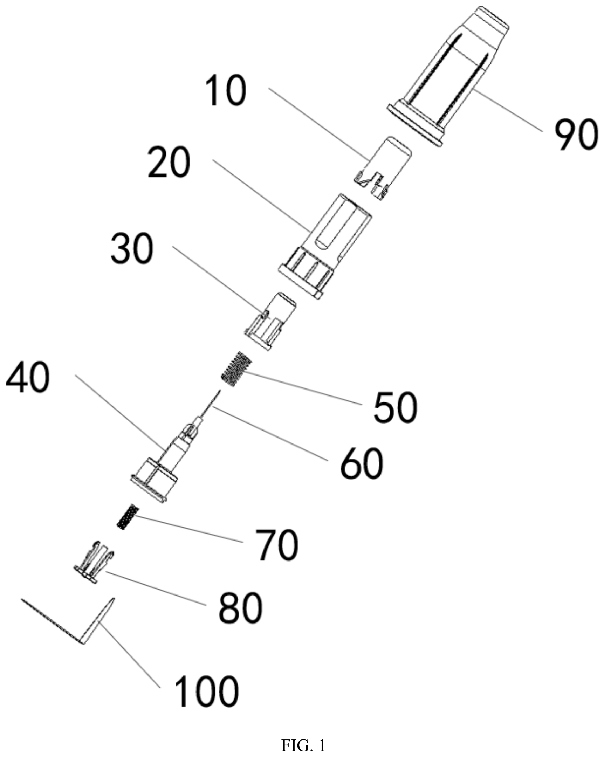

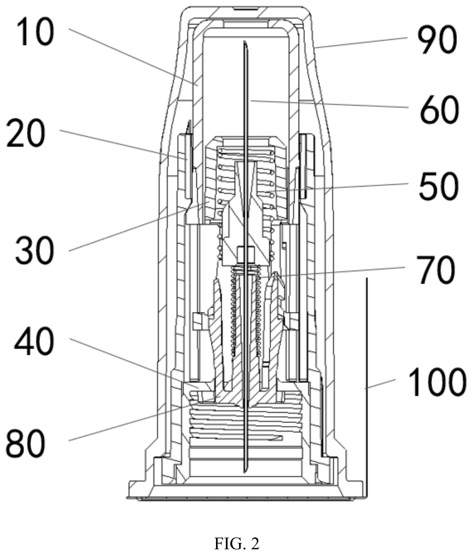

According to an exemplary embodiment of the present disclosure, as shown in FIGS. 1 and 2, a disposable injection needle includes an inner sheath 10, a shell 20, an inner core 30, a needle seat 40, a second spring 50, a needle tube 60, a first spring 70, a tail sheath 80, an outer cover 90, and a sealing label 100. The needle seat 40 is provided with the needle tube 60 extending axially therethrough.

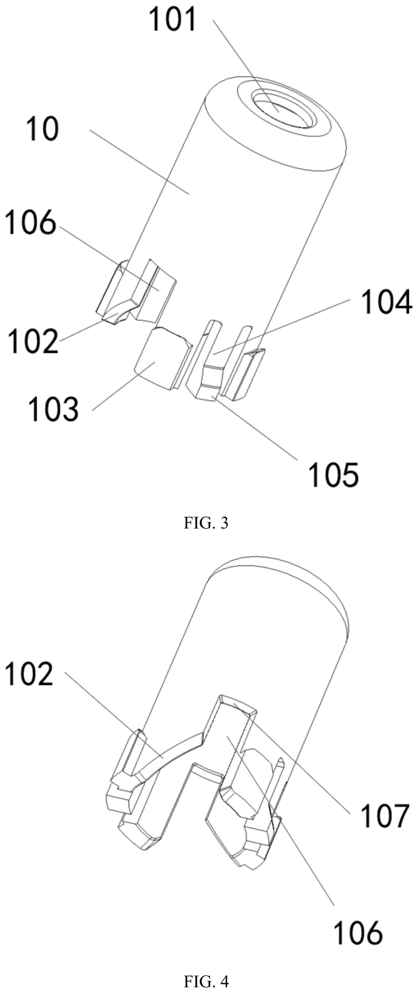

According to an exemplary embodiment of the present disclosure, as shown in FIGS. 3 and 4, the specific structure of the inner sheath 10 is illustrated.

One end of the inner sheath 10 has a first opening 101, while the other end of the inner sheath 10 is located within an accommodating space formed between the shell 20 and the needle seat 40. A portion of the inner sheath 10 is axially slidable within this accommodating space.

In this disclosure, one end or the first end of the inner sheath, i.e., the upper side of the inner sheath, is the side with the first opening 101; the other end or the second end, i.e., the lower side of the inner sheath.

The other end of the inner sheath 10 is provided with an inner sheath guide part 102. Optionally, the inner sheath guide part 102 is the end surface of the other end of the inner sheath 10.

In an optional embodiment, the inner sheath guide part 102 is a downwardly inclined slope at the other end of the inner sheath 10.

Optionally, a first guide groove 106 is provided adjacent to the inner sheath guide part 102, extending from the other end of the inner sheath 10 toward one end, wherein the top of the first guide groove 106 has a first blocking surface 107.

In an optional embodiment, an elastic arm 104 is provided at the other end of the inner sheath 10, and a radially outward protruding structure is provided at the free end of the elastic arm 104, forming an elastic arm end part 105.

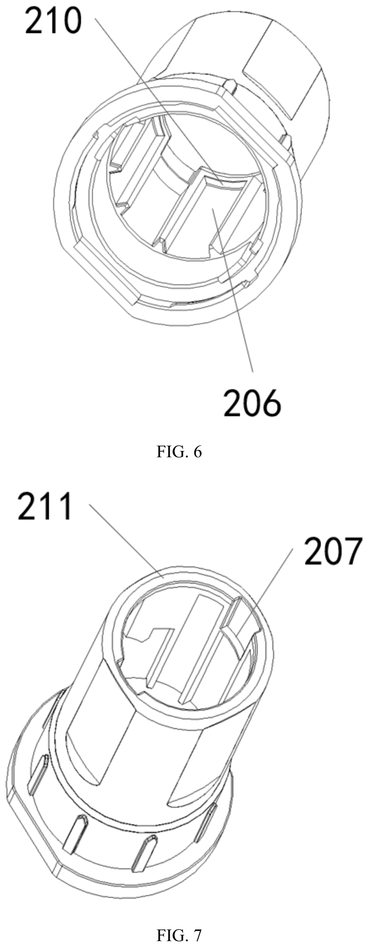

In an optional embodiment, a guide block 103 extending radially outward is provided on the outer wall of the other end of the inner sheath 10, and the inner wall of the shell 20 is provided with a fourth guide groove 206 extending in the axial direction. The guide block 103 moves along the guide groove 206 in the axial direction, so that the inner sheath 10 can only move axially along the fourth guide groove of the shell 20 and cannot rotate circumferentially. The guide block 103 can be positioned at any circumferential location on the inner sheath 10, and correspondingly, the fourth guide groove 206 is positioned at any location on the inner wall of the shell 20 for fitting with the guide block 103, as shown in FIGS. 3 and 6.

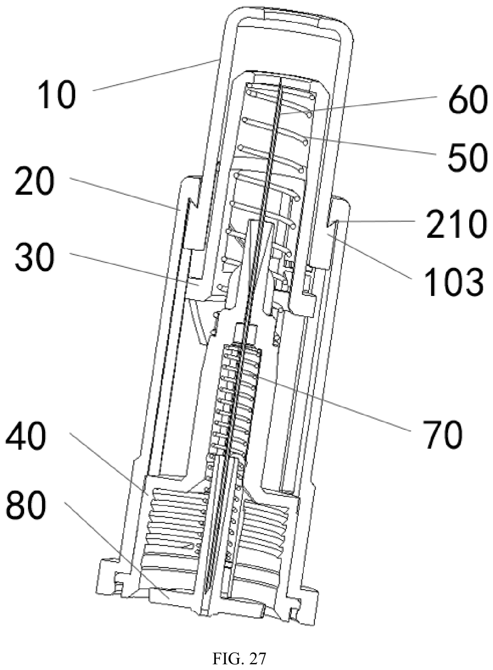

The upper end of the fourth guide groove 206 has a third blocking surface 210 that can prevent the guide block 103 from moving further upward along the fourth guide groove 206. Since the upper end of the fourth guide groove 206 has the third blocking surface 210, this surface can limit the highest position of the inner sheath 10 when moving upward, as shown in FIG. 27.

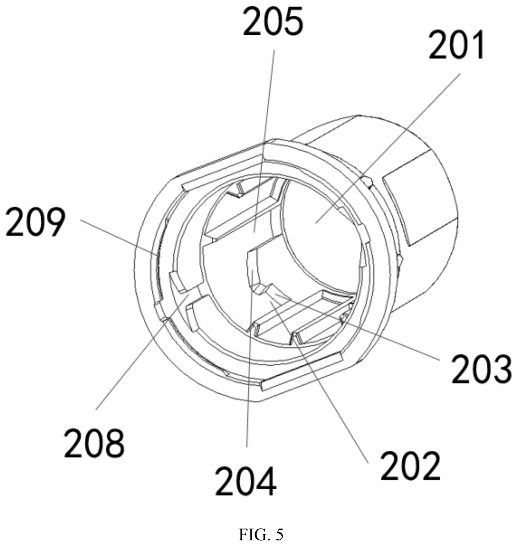

According to an exemplary embodiment of the present disclosure, as shown in FIGS. 5 to 7, the specific structure of the shell 20 is illustrated.

One end of the shell 20 has a second opening 201, and the other end of the shell 20 is connected to the other end of the needle seat 40, forming an accommodating space between the shell 20 and the needle seat 40.

The inner wall of the shell 20 is provided with a shell limit rib 204 extending in the axial direction, and a second guide groove 202 and a third guide groove 205 extending in the axial direction are respectively provided on both sides of the shell limit rib 204.

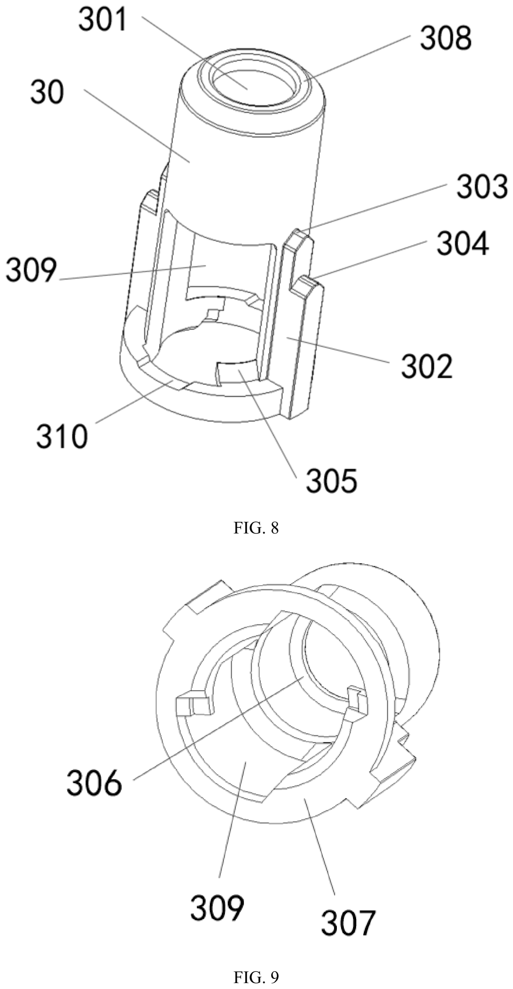

According to an exemplary embodiment of the present disclosure, as shown in FIGS. 8 and 9, the specific structure of the inner core 30 is illustrated.

As shown in FIG. 8, the front end of the inner core 30 has a third opening 301, and the outer wall of the inner core 30 is provided with an inner core guide part 302 that extends axially and protrudes radially outward.

Optionally, the inner core guide part 302 is a rib extending axially from the other end of the inner core 30 toward one end, where the other end is the bottom or lower part of the inner core 30.

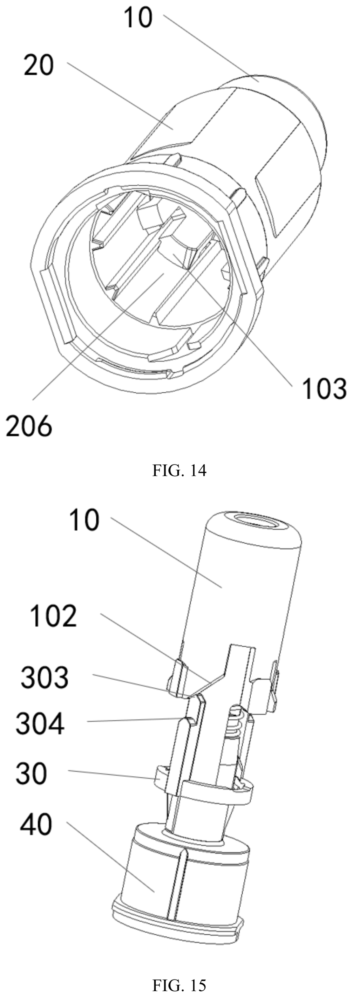

In an optional embodiment, the upper part of the inner core guide part 302 includes a first guide part 303 and a second guide part 304. Optionally, the first guide part 303 is an inclined slope that is fitted with the inner sheath guide part 102, meaning the slope where the first guide part 303 and the inner sheath guide part 102 contact each other is suitable for relative sliding, as shown in FIG. 15.

According to the disposable injection needle of the present disclosure, in the initial installation state, the top of the second guide part 304 is adapted to abut against the upper end surface of the second guide groove 202 of the shell 20, i.e., in contact with the second blocking surface 203, as shown in FIGS. 16 and 29.

Optionally, the distance between the top of the first guide part 303 and the bottom end surface of the inner core 307 is greater than the distance between the top of the second guide part 304 and the bottom end surface of the inner core 307, thereby forming a stepped structure in the upper part of the inner core guide part 302 due to the first guide part 303 and the second guide part 304, as shown in FIG. 8.

In an optional embodiment, there are two inner core guide parts 302, symmetrically arranged on the outer wall of the rear part of the inner core 30.

As shown in FIG. 15, at the installation position of the inner sheath 10 and the inner core 30, the first guide part 303 is fitted with the inner sheath guide part 102, maintaining a state of mutual contact.

As shown in FIGS. 16 and 29, at this point, the second guide part 304 is located within the second guide groove 202, and the second guide part 304 abuts against the second blocking surface 203 at the top of the second guide groove 202.

Since the side of the second guide part 304 is blocked by the shell limit rib 204, relative sliding in the circumferential direction between the first guide part 303 and the inner sheath guide part 102 is prevented.

Based on the external force pressing the inner sheath 10, the inner sheath 10 moves toward the accommodating space, and the inner core 30 moves downward together with the inner sheath 10 until the second guide part 304 reaches the bottom end surface position of the shell limit rib 204. At this point, the second guide part 304 is no longer blocked by the shell limit rib 204 in the circumferential direction. When the inner sheath 10 continues to be pressed downward, the first guide part 303 can slide along the inner sheath guide part 102, thereby guiding the inner core 30 to rotate in the circumferential direction. The first guide part 303 is adapted to move to be disengaged from the inner sheath guide part 102 and enter the first guide groove 106 until the top of the first guide part 303 abuts against the first blocking surface 107 at the top of the first guide groove 106, as shown in FIG. 21.

In optional embodiments, the shell limit rib 204 has two inclined slopes with a "V"-shaped structure at the bottom, and the second guide part 304 has corresponding two inclined mating surfaces with a triangular pyramid structure at the top. This allows the second guide part 304 to easily slide out of the circumferential limit of the shell limit rib 204 when moving downward to the bottom end of the shell limit rib 204, then continue sliding along another inclined mating surface of the shell limit rib 204 until disengaged.

Optionally, after the second guide part 304 is disengaged from the circumferential limit of the shell limit rib 204, under the downward external force, the first guide part 303 of the inner core 30 is adapted to slide along the inner sheath guide part 102. Meanwhile, the bottom slope of the second guide part 304 also slides along the corresponding bottom slope of the shell limit rib 204. Optionally, the inclination angle of the corresponding bottom slope of the shell limit rib 204 is the same as that of the slope of the inner sheath guide part 102.

Optionally, the inner core 30 is provided with inner core through slots 309 circumferentially on both sides of the inner core guide part 302. The bottom of the inner core 30 is equipped with a boss part 310 extending radially outward, and the boss part 310 is arranged circumferentially around the bottom of the inner core 30.

In optional embodiments, the inner core 30 is provided with an inner core engagement part 305 extending radially inward from the inner wall surface of the boss part 310.

Optionally, the inner core engagement part 305 is an upwardly inclined protrusion from the inner wall surface of the boss part 310, as shown in FIG. 20.

Optionally, one end of the inner core engagement part 305 is fixedly connected to the inner wall surface of the boss part 310, while the other end is a free end. The tip of the free end has an upwardly inclined engagement structure, as shown in FIG. 8.

In optional embodiments, the inner wall of the shell 20 is provided with a shell rib 207 protruding radially inward. Optionally, the shell rib 207 is a wedge-shaped or inverted triangular pyramid structure, with its upper part being a flat or step surface.

As shown in FIGS. 16 and 17, the elastic arm end part 105 of the inner sheath 10 is arranged opposite to the shell rib 207. In the initial installation position, the inner core 30 is in the first circumferential position, and the outer side of the elastic arm end part 105 faces the side of the shell rib 207. At this time, the shell 20, inner sheath 10, and inner core 30 are tightly fitted together, with the elastic arm end part 105 clamped between the shell rib 207 and the outer wall of the inner core 30, leaving no space for radial deformation of the elastic arm 105. The elastic arm 104 and its elastic arm end part 105 are in a naturally extended state.

After the inner core 30 rotates in the circumferential direction, it reaches the second circumferential position. The first guide part 303 of the inner core 30 enters the first guide groove 106 until the top end of the first guide part 303 contacts the first blocking surface 107 at the top of the first guide groove 106. Subsequently, the inner sheath 10 and the inner core 30 move downward together under external force. After the external force is removed, the inner sheath 10 and the inner core 30 move upward together due to the elastic force of the second spring 50.

During the rotation of the inner core 30, it not only moves circumferentially relative to the inner sheath 10 but also moves relative to inner sheath 10 in the axial direction, causing the elastic arm end part 105 of the inner sheath 10 to no longer be constrained by the outer wall surface of the inner core. As a result, the inner wall surface of the elastic arm end part 105 aligns with the inner core through slot 309 of the inner core 30. At this point, the inner core through slot 309 provides space for the elastic arm end part 105 to deform radially inward.

After the inner sheath 10 is fully pressed down, the bottom end surface of the inner core 307 contacts the needle seat limit surface 405.

After the external force is removed, the inner sheath 10 and the inner core 30 slide upward under the action of the second spring 50. When the elastic arm end part 105 of the inner sheath 10 contacts the shell rib 207 of the shell 20, since the inner core 30 has already rotated, the inner core through slot 309 aligns radially with the elastic arm end part 105. Under the spring force, the elastic arm 104 and elastic arm end part 105 of the inner sheath 10 deform radially inward and then slide upward over the shell rib 207 of the shell 20. After passing the shell rib 207, the elastic arm 104 and elastic arm end part 105 deform radially outward, returning to their natural state.

Subsequently, the inner sheath 10 and the inner core 30 continue moving outward from the accommodating space under the action of the second spring 50 until the top of the guide block 103 of the inner sheath 10 contacts the third blocking surface 210 at the top of the fourth guide groove 206 of the shell 20, stopping further upward movement.

In the second circumferential position, after the elastic arm end part 105 slides over the shell rib 207, the bottom end surface of the elastic arm end part 105 can abut against the top end surface of the shell rib 207. This prevents the inner sheath 10 from being pressed down again, as the elastic arm end part 105 is blocked by the shell rib 207, ensuring the needle tube 60 cannot protrude again from the first opening 101 of the inner sheath. Thus, the disclosed injection needle cannot be reused after initial use, avoiding accidental injuries and cross-contamination risks.

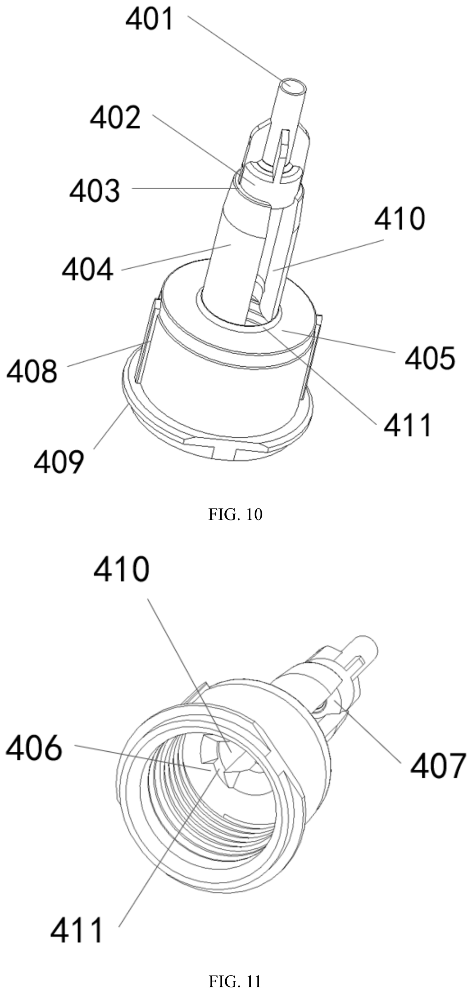

According to an exemplary embodiment of the present disclosure, as shown in FIGS. 10 and 11, the specific structure of the needle seat 40 is illustrated.

The needle seat 40 has a first end facing the blood collection site during blood collection and a second end away from the blood collection direction. Optionally, the first end and the second end are separated by the needle seat limit surface 405, meaning the end above the needle seat limit surface 405 is the first end, and the end below the needle seat limit surface 405 is the second end.

At the first end of the needle seat 40, the needle seat 40 has a small diameter part 402 and a large diameter part 404 connected to each other in the axial direction, forming the needle seat step surface 403 at the junction. The large diameter part 404 may be a hollow cylindrical-like structure.

One end of the second spring 50 is sleeved over the outer side of the small diameter part 402 and abuts against the needle seat step surface 403, as shown in FIG. 17.

The large diameter part of the needle seat 40 is provided with a needle seat through slot 410 extending from the first end of the large diameter part toward the other end.

At the position where the needle seat limit surface 405 contacts and wraps around the large diameter part 404, the needle seat limit surface 405 has a needle seat engagement part 411 inclined upward, as shown in FIGS. 24 and 25.

In an optional embodiment, as shown in FIG. 5, the bottom of the shell 20 is provided with a base, and the inner wall of the base is provided with a shell transverse slot 209 arranged circumferentially. Optionally, the inner wall of the rear end of the shell 20 is provided with a shell longitudinal slot 208 extending axially.

As shown in FIG. 10, the bottom of the needle seat 40 is provided with a needle seat transverse rib 409 and a needle seat longitudinal rib 408. The needle seat transverse rib 409 is arranged circumferentially on the outer wall of the needle seat 40, while the needle seat longitudinal rib 408 is arranged axially on the outer wall of the needle seat 40. When the disposable injection needle is assembled, the needle seat transverse rib 409 engages with the shell transverse slot 209 to fix the needle seat 40 and the shell 20 in the axial position. The needle seat longitudinal rib 408 engages with the shell longitudinal slot 208 to fix the needle seat 40 and the shell 20 in the circumferential position.

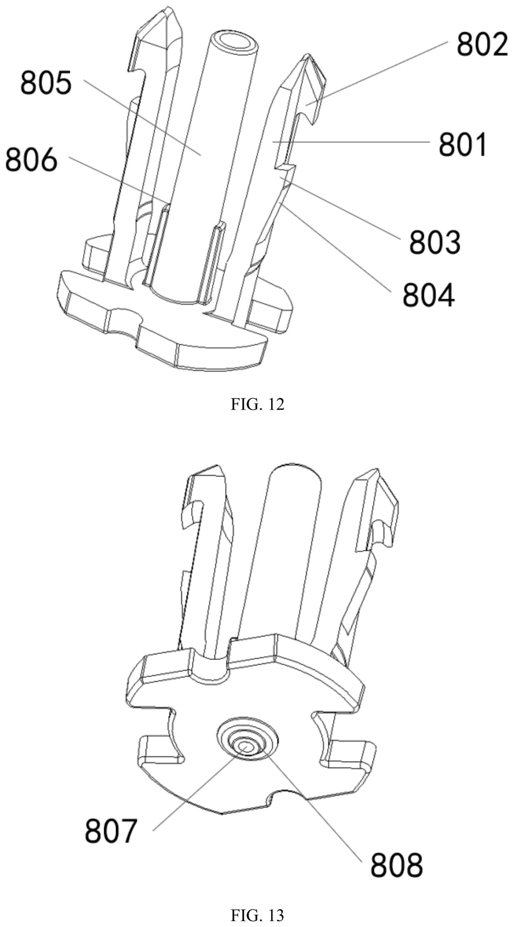

According to an exemplary embodiment of the present disclosure, as shown in FIGS. 12 and 13, the specific structure of the tail sheath 80 is illustrated.

The tail sheath 80 is arranged at the other end of the needle seat 40 and is provided with a tail sheath hole 807 for the rear end of the needle tube 60 to pass through. The tail sheath 80 includes an extension member 801 extending substantially along the direction of the needle tube. In the initial assembled state of the disposable injection needle, as shown in FIGS. 16, 18, and 19, the tail sheath 80 is positioned at the second end of the needle seat 40, and the needle tube 60 passes through the tail sheath hole 807. At this time, the tail sheath 80 is in the first axial position where the tail end of the needle tube 60 is exposed from the tail sheath hole 807, and the inner core 30 is in the first circumferential position engaged with the extension member 302. When the disposable injection needle is used, after an external force is applied to the disposable injection needle, the inner core 30 rotates from the first circumferential position fitted with the extension member 801 to the second circumferential position disengaged from the extension member 801.

As the inner core 30 rotates from the first circumferential position to the second circumferential position, the tail sheath 80 moves from the first axial position toward the second axial position under the action of the first spring 70. When the inner core 30 rotates to the second circumferential position, it is disengaged from the extension member 801. At this point, the tail sheath 80 moves to the second axial position under the elastic force of the first spring 70 to cover the tail end of the needle tube 60, as shown in FIGS. 23 to 25, thereby preventing the needle tube 60 from protruding from the tail end of the injection needle and causing injury or infection.

Optionally, the extending end of the extension member 801 has a tail sheath engagement part 802, which is adapted to pass through the needle seat through slot 410 and be engaged with the inner core engagement part 305 of the inner core 30 to maintain the tail sheath 80 in the first axial position, as shown in FIGS. 16, 18, and 19. That is, the tail sheath 80 is in the first axial position where the tail end of the needle tube 60 is exposed from the tail sheath hole 807, and the inner core 30 is in the first circumferential position fitted with the extension member 302. At this time, as shown in FIG. 19, the tail sheath engagement part 802 of the extension member 801 of the tail sheath 80 engages with the inner core engagement part 305 of the inner core 30. When the disposable injection needle is used, after an external force is applied to the disposable injection needle, the inner core 30 rotates from the first circumferential position fitted with the tail sheath engagement part 802 of the extension member 801 to the second circumferential position disengaged from the tail sheath engagement part 802. When the inner core 30 is in the second circumferential position, the tail sheath engagement part 802 of the extension member 801 is disengaged from the inner core engagement part 305 of the inner core 30.

In an optional embodiment, the extension member 801 is further provided with a clamping and fitting part 803, as shown in FIG. 12. Based on the movement of the tail sheath 80 from the first axial position toward the second axial position, the clamping and fitting part 803 is adapted to move from one side of the needle seat limit surface 405 to the other side, as shown in FIGS. 24 and 25.

According to an embodiment of the present disclosure, at the first axial position of the tail sheath 80, the extension member 801 extends past the needle seat limit surface 405 to be engaged with the inner core engagement part 305 of the inner core 30; at the second axial position of the tail sheath 80, the extension member 801 is adapted to be engaged with the needle seat limit surface 405.

Optionally, the extension member 801 is an elastic member. When the clamping and fitting part 803 moves from one side of the needle seat limit surface 405 toward the other side, the clamping and fitting inclined surface 804 of the clamping and fitting part 803 abuts against the needle seat engagement part 411 of the needle seat limit surface 405 and continues to move toward the tail end of the injection needle under the thrust of the first spring 70. Since the extension member 801 is elastic, the clamping and fitting part 803 moves radially inward to pass over the needle seat engagement part 411 and move to the other side of the needle seat limit surface 405.

At this point, as shown in FIGS. 23 to 28, the bottom surface 808 of the tail sheath 80 is closer to the tail end of the injection needle than the tail end of the needle tube 60, and since the needle seat engagement part 411 is located between the tail sheath engagement part 802 of the extension member 801 and the clamping and fitting part 803 and cannot be pressed down, the tail end of the needle tube 60 cannot protrude from the tail sheath hole 807, thereby protecting the user from accidental needle stick injuries.

In an optional embodiment, the tail sheath 80 has a central columnar body 805 extending axially to allow the needle tube 60 to pass through, and a tail sheath step surface 806 is formed at the bottom of the central columnar body. One end of the first spring 70 is sleeved on the outside of the central columnar body 805 and abuts against the tail sheath step surface 806, while the other end of the first spring 70 abuts the small diameter bottom end surface 407 of the needle seat 40.

In an optional embodiment, the elastic force of the first spring 70 is less than that of the second spring 50, so that in the initial installation state of the disposable injection needle, the inner core 30 remains relatively stable due to the elastic force of the second spring 50.

Below, the assembly process of the disposable injection needle according to an exemplary embodiment of the present disclosure is described exemplarily with reference to the above structure.

The front end of the inner sheath 10 passes through the second opening 201 of the shell 20. The guide block 103 of the inner sheath 10 is fitted with the fourth guide groove 206 of the shell 20, allowing the inner sheath 10 to move axially relative to the shell 20 without circumferential rotation, as shown in FIG. 14.

The needle tube 60 passes through the needle seat hole 401 and is fixed inside the needle seat hole 401 with adhesive.

The front end of the inner core 30 is located within the cavity of the inner sheath 10. Under the upward force exerted by the second spring 50 on the inner end surface of the inner core 30's front end or the inner core blocking surface 306, the first guide part 303 contacts the inner sheath guide part 102, and the second guide part 304 of the inner core 30 contacts the second blocking surface 203 at the top of the second guide groove 202 of the shell 20, as shown in FIGS. 15 and 16.

The shell rib 207 of the shell 20 restricts the upward movement of the elastic arm end part 105 of the inner sheath 10, keeping the inner sheath 10 within a limited position, as shown in FIG. 16.

One end of the second spring 50 contacts the inner core blocking surface 306, while the other end contacts the needle seat step surface 403, as shown in FIG. 17.

The needle seat 40, the inner core 30, the second spring 50 and the needle tube 60 are assembled within the cavity of the shell 20. The needle seat longitudinal rib 408 of the needle seat 40 is fitted with the shell longitudinal slot 208 of the shell, preventing circumferential rotation between the needle seat 40 and the shell 20. The needle seat transverse rib 409 is fitted with the shell transverse slot 209, fixing the axial position of the needle seat 40 relative to the shell 20.

The tail sheath 80 is placed at the rear end of the needle seat 40. The tail sheath hole 807 passes through the rear end of the needle tube 60. The extension member 801 of the tail sheath 80 passes through the needle seat through slot 410. The tail sheath engagement part 802 of the extension member 801 of the tail sheath 80 is engaged with the inner core engagement part 305. The first spring 70 is positioned between the tail sheath 80 and the needle seat 40, with the first spring 70 sleeved around the outside of the central columnar body 805 of the tail sheath 80. One end of the first spring 70 contacts the tail sheath step surface 806 of the tail sheath 80, while the other end contacts the small diameter bottom end surface 407 of the needle seat 40, as shown in FIG. 19.

The internal assembly, consisting of the inner sheath 10, the shell 20, the inner core 30, the needle seat 40, the second spring 50, the needle tube 60, the first spring 70 and the tail sheath 80, is placed within the cavity of the outer cover 90. The sealing label 10 seals the bottom opening of the outer cover 90.

Below, the usage process of the disposable injection needle according to the exemplary embodiment of the present disclosure is described with reference to the above structure.

Before use, tear off the sealing label 10 and remove the outer cover 90 to take out the internal components.

At this point, the states of each component are as shown in FIGS. 16, 17, 18, and 19: under the action of the second spring 50, the first guide part 303 contacts the inner sheath guide part 102, and the second guide part 304 contacts the second blocking surface 203 of the shell 20, as illustrated in FIGS. 15 and 16.

One end of the second spring 50 contacts the inner core blocking surface 306, while the other end contacts the needle seat step surface 403, as shown in FIG. 17.

The shell rib 207 of the shell 20 restricts the upward movement of the elastic arm end part 105 of the inner sheath 10, keeping the inner sheath 10 within the defined position, as shown in FIG. 16.

The position of the needle seat 40 is relatively fixed to the shell 20.

The tail sheath 80 is placed at the rear end of the needle seat 40, with the tail sheath hole 807 passing through the rear end of the needle tube 60. The extension member 801 of the tail sheath 80 passes through the needle seat through slot 410, and the tail sheath engagement part 802 on the extension member 801 of the tail sheath 80 engages with the inner core engagement part 305. The first spring 70 is positioned between the tail sheath 80 and the needle seat 40, assembled around the outer side of the central columnar body 805 of the tail sheath 80. One end of the first spring 70 contacts the tail sheath step surface 806 of the tail sheath 80, while the other end contacts the small diameter bottom end surface 407 of the needle seat 40, as shown in FIG. 19.

Below, the operation process of the disposable injection needle according to the exemplary embodiment of the present disclosure is described with reference to the above structure.

When using the disposable injection needle, the inner sheath 10 moves toward the needle seat 40 under external force. At this time, the inner sheath guide part 102 pushes the first guide part 303 of the inner core 30, causing the inner core 30 to move together toward the needle seat 40 until the second guide part 304 of the inner core 30 is disengaged from the second guide groove 202 of the shell 20.

In the initial state, the first guide part 303 on the inner core 30 abuts against the inner sheath guide part 102, as shown in FIG. 15. The second guide part 304 on the inner core 30 is located within the second guide groove 202 of the shell 20, with the upper end surface of the second guide part 304 abutting against the step surface at the top of the second guide groove, i.e., the second blocking surface 203, as shown in FIGS. 5, 16, and 29.

When the inner sheath 10 is subjected to external force, it pushes the inner core 30 to move together toward the end of the needle seat until the second guide part 304 reaches the bottom end of the shell limit rib 204. At this point, the second guide part 304 is no longer circumferentially restricted by the shell limit rib 204.

Subsequently, under the upward force applied by the second spring 50, the first guide part 303 slides relative to the inner sheath guide part 102, causing the inner core 30 to rotate circumferentially until the first guide part 303 contacts the first blocking surface 107 of the inner sheath 10, as shown in FIG. 21.

During this process, the inner core engagement part 305 undergoes circumferential displacement relative to the tail sheath engagement part 802 on the extension member 801 of the tail sheath 80 and is disengaged from the hooked state, as shown in FIG. 20.

As the inner core engagement part 305 undergoes circumferential displacement relative to the tail sheath engagement part 802 and disengages, the tail sheath 80 moves toward the distal end under the action of the first spring 70 until the tail sheath engagement part 802 contacts the needle seat limit surface 405 of the needle seat 40. At this point, the tail sheath engagement part 802 is engaged with the needle seat engagement part 411 of the needle seat 40, as shown in FIGS. 23 and 24.

During this movement, when the clamping and fitting inclined surface 804 of the clamping and fitting part 803 of the tail sheath 80 contacts the needle seat engagement part 411, the extension member 801 of the tail sheath 80 deforms radially inward until the clamping and fitting part 803 slides past the needle seat engagement part 411. Afterward, the extension member 801 of the tail sheath 80 returns to its initial state.

If pressure is applied to the tail sheath 80 toward the proximal end at this stage, the clamping and fitting part 803 of the tail sheath 80 will interfere with the bottom surface 406 of the needle seat engagement part 411, preventing the tail sheath from being pressed. The tail sheath bottom surface 808 is positioned farther toward the distal end relative to the tail end of the needle tube 60, ensuring the tail end of the needle tube 60 cannot protrude from the tail sheath hole 807, thereby protecting the user. Additionally, once the tail sheath 80 pops out, in the state shown in FIGS. 23 and 24, the disposable injection needle cannot reconnect to the insulin injection pen, rendering it unusable again.

If the disposable injection needle is assembled on an insulin injection pen during use, the tail sheath 80 cannot pop out due to obstruction by the pen's structure. Only after removing the disposable injection needle from the insulin pen will the tail sheath 80 fully extend to the state shown in FIGS. 23 and 24.

Under external force, the inner sheath 10 continues moving toward the needle seat 40 until the inner core 30 contacts the needle seat 40. At this point, the bottom end surface of the inner core 307 contacts the needle seat limit surface 405, and the inner sheath 10 stops moving. During this movement, the needle tube 60 is exposed from the first opening 102 of the inner sheath 10, as shown in FIGS. 21 and 22.

After the external force is released, the inner sheath 10 and inner core 30 slide upward together under the action of the second spring 50, with the second guide part 304 of the inner core 30 sliding into the third guide groove 205 of the shell 20, as shown in FIG. 5.

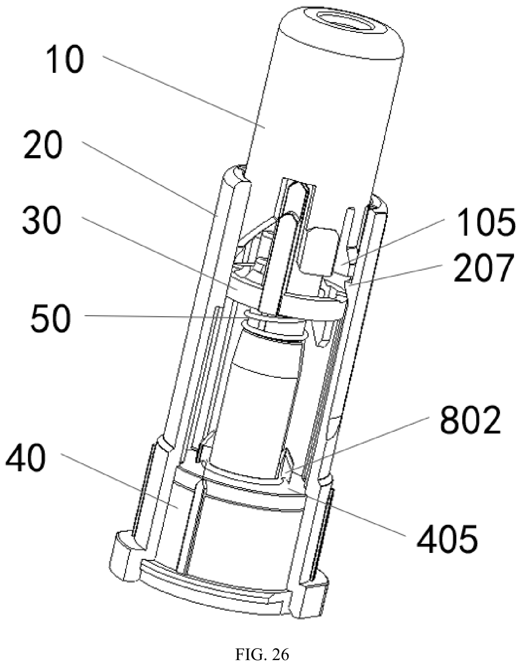

When the elastic arm end part 105 of the inner sheath 10 contacts the shell rib 207 of the shell 20, under the spring force, the elastic arm 104 of the inner sheath 10 deforms radially inward, causing the elastic arm end part 105 of the inner sheath 10 to slide over the shell rib 207. After sliding over the shell rib 207, the elastic arm 104 and elastic arm end part 105 of the inner sheath 10 return radially outward to their natural state. As shown in FIGS. 25 and 26.

The inner sheath 10 and inner core 30 continue to move upward under the action of the second spring 50 until the top of the guide block 103 of the inner sheath 10 contacts the third blocking surface 210 of the shell 20, stopping the movement, as shown in FIG. 27.

At this point, if pressure is applied downward on the inner sheath 10 again, interference occurs between the elastic arm end part 105 of the inner sheath 10 and the shell rib 207, preventing further downward movement and ensuring the needle tube 60 cannot re-emerge from the first opening 101 of the inner sheath 10, as shown in FIG. 28.

In the disposable injection needle according to this disclosure, before use, the inner core front end surface 308 may be higher or lower than the shell front end surface 211; after use, the inner core front end surface 308 is higher than the shell front end surface 211. In this embodiment, before use, the inner core front end surface 308 is lower than the shell front end surface 211, and after use, the inner core front end surface 308 is higher than the shell front end surface 211. When the inner sheath 10 is made of transparent or semi-transparent material, this difference can be used to distinguish whether the disposable injection needle is in a pre-use or post-use state.

It should be noted that the above technical solutions can be combined in any logical manner, all falling within the scope of this disclosure. In this document, relational terms such as "first" and "second" are used solely to distinguish one entity or operation from another, without necessarily implying any actual relationship or order between them. Furthermore, terms like "comprising," "including," or any variations thereof are intended to cover non-exclusive inclusion, meaning that a process, method, article, or device comprising a list of elements includes not only those elements but also other elements not explicitly listed or inherent to such process, method, article, or device. Without further limitation, an element defined by the phrase "comprising a..." does not exclude the presence of additional identical elements in the process, method, article, or device.

Each embodiment in this specification is described in a related manner, with identical or similar parts cross-referenced between embodiments. Each embodiment primarily highlights its differences from others.

Although embodiments of the present disclosure have been shown and described, it should be understood by those of ordinary skill in the art that changes and combinations of elements can be made to these embodiments without departing from the principles and spirit of the disclosure. The scope of the disclosure is defined by the appended claims and their equivalents.

## Claims

1. A disposable injection needle, comprising:
a needle seat (40) provided with a needle tube (60) extending axially therethrough and having a first end and a second end;
a shell (20), a second end of the shell (20) being connected to the second end of the needle seat (40) to form an accommodating space between the shell (20) and the needle seat (40), and a first end of the shell (20) having a second opening (201);
an inner sheath (10), a second end of the inner sheath (10) being located within the accommodating space, a first end of the inner sheath (10) having a first opening (101) and being adapted to extend out of the second opening (201), and a portion of the inner sheath (10) being axially slidable within the accommodating space;
an inner core (30), a second end of the inner sheath (10) being sleeved on a first end of the inner core (30), wherein the first end of the inner core (30) comprises a third opening (301) for the first end of the needle tube (60) to extend through;
a second spring (50) disposed between the inner core (30) and the first end of the needle seat (40) and adapted to provide an elastic force for axially moving the inner core (30) and the inner sheath (10) together outward from the accommodating space;
a tail sheath (80) disposed at the second end of the needle seat (40) and having a tail sheath hole (807) for the needle tube (60) to pass through, wherein the tail sheath (80) comprises an extension member (801) extending substantially in an axial direction, and the tail sheath (80) has a first axial position and a second axial position along the axial extension direction, wherein in the first axial position, the second end of the needle tube (60) is exposed from the tail sheath hole (807), and in the second axial position, the second end of the needle tube (60) is not exposed from the tail sheath hole (807);
a first spring (70) disposed between the needle seat (40) and the tail sheath (80) to provide power for the tail sheath (80) to move from the first axial position toward the second axial position;
wherein:
the inner core (30) has a first circumferential position and a second circumferential position, wherein before the injection needle is used, the inner core (30) is in the first circumferential position where the inner core (30) fittingly abuts against the extension member (801) in the axial direction, and after the injection needle is used, the inner core (30) is in the second circumferential position where the inner core (30) is disengaged from the extension member (801) in the axial direction; and
based on the inner core (30) rotating from the first circumferential position to the second circumferential position, the tail sheath (80) is adapted to move from the first axial position toward the second axial position under the action of the first spring (70).

2. The disposable injection needle according to claim 1, wherein:
the second end of the inner core (30) is provided with a radially inward-extending inner core engagement part (305), and an extending end of the extension member (801) is provided with a tail sheath engagement part (802); and
in the first axial position of the tail sheath (80), the tail sheath engagement part (802) is adapted to be engaged with the inner core engagement part (305).

3. The disposable injection needle according to claim 2, wherein:
the second end of the inner core (30) is provided with an annular boss part (310) arranged along a circumferential direction, and the inner core engagement part (305) extends radially inward from an inner wall surface of the boss part (310); optionally,
one end of the inner core engagement part (305) is fixedly connected to the inner wall surface of the boss part (310), and the other end of the inner core engagement part (305) is a free end with an upwardly inclined engagement structure.

4. The disposable injection needle according to claim 2, wherein:
the needle seat (40) has a needle seat limit surface (405), and the first end and the second end of the needle seat (40) are located on opposite sides of the needle seat limit surface (405), respectively; and
a side wall of the first end of the needle seat (40) is provided with an axially extending needle seat through slot (410), and the tail sheath engagement part (802) of the extension member (801) is adapted to extend through the needle seat limit surface (405) and protrude from the needle seat through slot (410) to be engaged with the inner core engagement part (305), thereby maintaining the tail sheath (80) in the first axial position.

5. The disposable injection needle according to claim 4, wherein:
the tail sheath (80) comprises a plurality of extension members (801), and the plurality of extension members (801) have a same height in the axial extension direction; optionally,
the extension member (801) further has a clamping and fitting part (803), and based on the tail sheath (80) moving from the first axial position toward the second axial position, the clamping and fitting part (803) is adapted to move from one side of the needle seat limit surface (405) to the other side of the needle seat limit surface (405).

6. The disposable injection needle according to claim 1, wherein:
the tail sheath (80) is further provided with an axially extending central columnar body (805), and the needle tube (60) passes through the central columnar body (805); and
a side wall at one end of the central columnar body (805) is provided with a radially outwardly extending tail sheath step surface (806), and one end of the first spring (70) is sleeved on the central columnar body (805) and abuts against the tail sheath step surface (806).

7. The disposable injection needle according to claim 1, wherein:
an inner wall of the shell (20) is provided with a radially inwardly protruding shell rib (207), and the inner sheath (10) is provided with an elastic arm (104) that is fitted with the shell rib (207);
in the second circumferential position, the second spring (50) is adapted to push the inner core (30) and the inner sheath (10) to move together away from the accommodating space; and
during the movement, the elastic arm (104) is adapted to pass over the shell rib (207), and the shell rib (207) is adapted to prevent the elastic arm (104) that has passed over the shell rib (207) from moving further in a direction toward the accommodating space.

8. The disposable injection needle according to claim 7, wherein:
the shell rib (207) is a wedge-shaped structure or an inverted triangular pyramid structure, with one end thereof having a flat surface or step surface;
the free end of the elastic arm (104) has a radially outward protruding elastic arm end part (105); and
at the first circumferential position, the elastic arm end part (105) is adapted to be arranged opposite to the shell rib (207); and at the second circumferential position, the elastic arm end part (105) is adapted to abut against the flat surface or step surface above the shell rib (207).

9. The disposable injection needle according to claim 8, wherein:
a side wall of the inner core (30) is provided with an inner core through slot (309);
at the first circumferential position of the inner core (30), the elastic arm end part (105) is arranged opposite to the side wall of the inner core (30); and at the second circumferential position of the inner core (30), the elastic arm end part (105) is arranged opposite to the inner core through slot (309).

10. The disposable injection needle according to claim 1, wherein:
one end of the inner sheath (10) is provided with an inner sheath guide part (102), and an outer wall of the inner core (30) is provided with a first guide part (303) protruding radially outward; and at an installation position of the inner sheath (10) and the inner core (30), the inner sheath guide part (102) is fitted with the first guide part (303); and
based on the movement of the inner sheath (10) toward the accommodating space, the first guide part (303) is adapted to move along the inner sheath guide part (102) to guide the rotation of the inner core (30) in the circumferential direction and is adapted to move to be disengaged from the inner sheath guide part (102).

11. The disposable injection needle according to claim 10, wherein:
the inner wall of the shell (20) is provided with a shell limit rib (204) extending in the axial direction, and a second guide groove (202) and a third guide groove (205) extending in the axial direction are respectively provided on both sides of the shell limit rib (204);
the inner core (30) is further provided with a second guide part (304) extending radially outward;
at the installation position of the inner sheath (10) and the inner core (30), the second guide part (304) is located in the second guide groove (202); and
based on the movement of the inner sheath (10) toward the accommodating space and the rotation of the inner core (30) in the circumferential direction, the second guide part (304) is adapted to pass over the shell limit rib (204) and enter the third guide groove (205).

12. The disposable injection needle according to claim 11, wherein:
the second guide part (304) is located radially outward of the first guide part (303), and a distance between a top end of the first guide part (303) and a bottom end surface of the inner core (307) is greater than a distance between a top end of the second guide part (304) and the bottom end surface of the inner core (307); optionally,
the shell limit rib (204) has two inclined slopes and has a "V"-shaped structure at a bottom thereof, and the second guide part (304) has two corresponding inclined mating surfaces and has a triangular pyramid structure at a top thereof,
such that the second guide part (304) is capable of continually sliding along the inclined slopes of the shell limit rib (204) after sliding over the shell limit rib (204).

13. The disposable injection needle according to claim 1, wherein:
the inner wall of the shell (20) is provided with a fourth guide groove (206) extending in the axial direction, and an outer wall of the inner sheath (10) is provided with a guide block (103) extending radially outward; and
the guide block (103) is adapted to move along the fourth guide groove (206) in the axial direction.

14. The disposable injection needle according to claim 13, wherein:
a top of the fourth guide groove (206) has a third blocking surface (210), and a top end of the guide block (103) is adapted to abut against the third blocking surface (210) to prevent the inner sheath (10) from further moving outward from the accommodating space.

15. The disposable injection needle according to claim 10, wherein:
the inner sheath (10) is provided with a first guide groove (106) extending in the axial direction, and based on the rotation of the inner core (30) in the circumferential direction, the first guide part (303) of the inner core (30) is adapted to enter the first guide groove (106); optionally,
at the installation position of the inner sheath (10) and the inner core (30), a inner core front end surface (308) is lower in height in the axial direction than a shell front end surface (211); and
after the injection needle is used, the inner core front end surface (308) is higher in height in the axial direction than the shell front end surface (211); optionally,
the inner wall of the shell (20) is provided with a shell transverse slot (209) and a shell longitudinal slot (208), and an outer side of the needle seat (40) is correspondingly provided with a needle seat transverse rib (409) and a needle seat longitudinal rib (408), so that the needle seat (40) and the shell (20) are fixed circumferentially and axially.
